(19) Europäisches Patentamt
European Patent Office
Office européen des brevets

(11) **EP 0 861 687 B1**

(12) **EUROPÄISCHE PATENTSCHRIFT**

(45) Veröffentlichungstag und Bekanntmachung des Hinweises auf die Patenterteilung:
**05.08.2009 Patentblatt 2009/32**

(51) Int Cl.:
***B01J 19/00*** *(2006.01)*    ***G01N 33/49*** *(2006.01)*
***G01N 21/27*** *(2006.01)*

(21) Anmeldenummer: **98101330.3**

(22) Anmeldetag: **27.01.1998**

(54) **Verfahren zur Auswertung von Reaktionsverläufen**

Method to evaluate reaction courses

Méthode d'évaluation des déroulements de réactions

(84) Benannte Vertragsstaaten:
**AT BE CH DE ES FR GB IT LI LU NL SE**

(30) Priorität: **27.02.1997 DE 19707897**

(43) Veröffentlichungstag der Anmeldung:
**02.09.1998 Patentblatt 1998/36**

(73) Patentinhaber: **Siemens Healthcare Diagnostics Products GmbH**
**35041 Marburg (DE)**

(72) Erfinder: **Meyers, Wilfried, Dr.**
**35037 Marburg (DE)**

(56) Entgegenhaltungen:
**US-A- 3 635 678        US-A- 4 454 752**
**US-A- 4 492 462        US-A- 4 822 568**

• **KOLDE H.J.: "ChromoTimeSystem-A New Generation of Coagulation Analyses" BEHRING INST.MITT., Bd. 78, 1985, DE, Seiten 176-187, XP002061515**

EP 0 861 687 B1

Printed by Jouve, 75001 PARIS (FR)

**Beschreibung**

Die Erfindung betrifft ein Verfahren zur störungsfreien Auswertung von Reaktionskinetiken, die nach der sogenannten Schwellenwertmethode auszuwerten sind. Solche Kurvenverläufe entstehen immer dann, wenn unter kontrollierten Umgebungsbedingungen der Übergang einer reaktionsabhängigen Meßgröße von einem Anfangszustand in einen Endzustand im zeitlichen Verlauf beobachtet wird.

Die Schwellenwertmethode ist ein in der Praxis häufig angewandtes Verfahren. Ein solches Verfahren wird z. B. von KOLDE (Behring Inst. Mitt. 78, S. 176 - 187 (1985)) beschrieben.

Beschrieben wurde schon die Charakterisierung eines Meßobjektes auf der Basis eines vorgegebenen Meßsystems durch ein 2-Schrittverfahren. Dabei wird

a) durch Zugabe gewisser Aktivierungskomponenten in dem Meßobjekt ein Prozeß in Gang gesetzt, welcher auf der Basis eines definierten Detektionsprinzips ein Signal im zeitlichen Verlauf liefert. Dies ergibt eine Meßreihe $(T_i, S_i), i = 1, ..., n$. Dabei bezeichnet $T_i$ den i-ten Zeitpunkt und $S_i$ das Signal zum Zeitpunkt $T_i$.

b) Die Meßreihe $(T_i, S_i), i = 1, ..., n$ wird mit Hilfe einer Transformation $f$ zu einem Rohwert R verdichtet:

$$(T_i, S_i), i = 1, \ldots, n \xrightarrow{\ f\ } R$$

Die Schwellenwertmethode ist eine solche Transformation $f$, wie sie in Schritt b) benötigt wird. Sie ist im wesentlichen charakterisiert durch einen Parameter $d$, welcher die tatsächlich anzuwendende Schwelle festlegt. Der Rohwert wird dabei als der Zeitpunkt definiert, zu dem ausgehend von einem Basiswert die Meßreihe erst malig die Schwelle nachhaltig überschreitet.

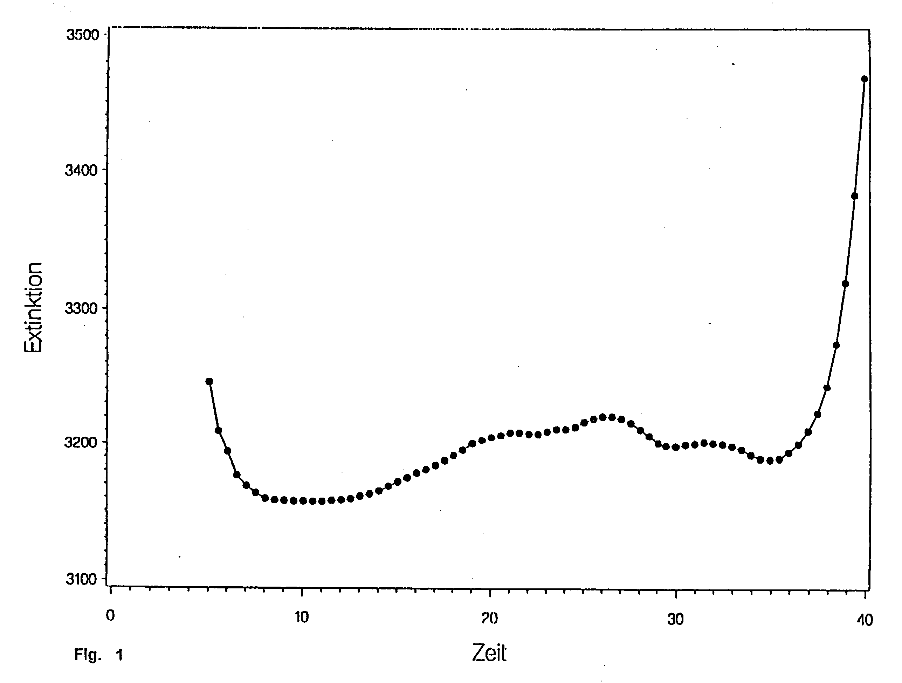

Das bekannte Verfahren ist nicht in der Lage, Kurvenverläufe, die einen wie in Fig. 1 dargestellten welligen Verlauf aufweisen, korrekt auszuwerten. In dem dort angegebenen Beispiel wäre der korrekte Rohwert ca. 38 sec. (nachhaltige Überschreitung einer vorgegebenen Schwelle) gewesen, tatsächlich wurden jedoch nach dem bekannten Verfahren ca. 25 sec. ermittelt. Eine solche Fehlbestimmung kann je nach Anwendungsgebiet zu schwerwiegenden Konsequenzen und letztlich sogar zu lebensgefährdenden Zuständen führen (siehe Beispiele ).

Der vorliegenden Erfindung lag also die Aufgabe zugrunde, ein Verfahren zur Verfügung zu stellen, das die obengenannten Nachteile eliminiert. Dies bedeutet, daß Fehlbestimmungen, wie sie nach dem beschriebenen Verfahren auftreten können, mit der technisch machbaren Wahrscheinlichkeit ausgeschlossen werden können.

Die Lösung dieses Problems erfolgt durch die Bereitstellung der in den Patentansprüchen beschriebenen Ausführungsformen. Es wurde überraschenderweise festgestellt, daß das Problem durch eine Änderung des Schrittes b) in dem vorher beschriebenen Verfahren gelöst werden kann. Der dort beschriebene Schritt, nämlich <u>einen</u> festen Operator $f$ auf die Meßreihe anzuwenden, ist zu ersetzen durch eine Folge von Schritten, bei denen stets ein anderer Operator zu verwenden ist und die so erhaltenen Rohwerte einem Plausibilitätscheck unterworfen werden. Der Austausch der Operatoren und die dadurch erfolgende korrekte Bewertung der Operatoren erfolgt automatisch durch die Datenverarbeitung des Meßsystems anhand vorher festgelegter, spezifischer Kenngrößen. Diese Kenngrößen sind geräte- und reaktionsspezifisch und können vom Fachmann durch einfache Vorversuche bestimmt werden. Überraschenderweise wurde festgestellt, daß folgende Konfiguration besonders vorteilhaft ist, da sie für viele Geräte-/ Reaktionskominationen anwendbar ist:

| | |
|---|---|
| $d_{max}$ | = 1. 5 bis 10 x $d_0$, bevorzugterweise etwa 2 x $d_0$ |
| n | = 5 bis 30, bevorzugterweise etwa 10 |
| $Q_0$ | = 5 bis 50, bevorzugterweise 15 bis 30, besonders bevorzugterweise etwa 20. |

Lediglich $d_0$ ist dabei noch direkt von dem Meßsystem abhängig.

Das Verfahren kann z. B. wie folgt durchgeführt werden:

a) Es werden für die Reaktion spezifische Werte für einen initialen Wert $d_0$, für einen maximalen Wert $d_{max}$ (> $d_0$), sowie eine Schrittzahl n und dadurch eine Schrittweite $d_s = (d_{max} - d_0)/n$ festgelegt;

b) für den Bereich von $d = d_0$ bis $d_{max}$ wird jeweils für $d_n = (d_0 + (d_s)*n)$ der Wert $T_n$ bestimmt,

c) aus der geordneten Reihe der Werte $T_0$ bis $T_{max}$ werden die Differenzen $D_i = T_i - T_{i-1}$ für $i = 1, ..., n$ gebildet und aus der Menge $D_1, ..., D_n$ der größte Wert $D_{max}$ und der kleinste Wert $D_{min}$ bestimmt, sowie der Quotient $Q = D_{max}/D_{min}$ gebildet,

d) nur Messungen, für die $Q \leq Q_0$ gilt werden zur weiteren Auswertung verwendet, Messungen für die $Q > Q_0$ gilt werden als zu wellig verworfen.

Die in diesem Verfahren einzustellenden Kenngrößen sind die folgenden:

| | |
|---|---|
| $d_0$ - | initiale Schwelle |
| $d_{max}$ - | maximale Schwelle |
| $n$ - | Schrittzahl (ganze Zahl > 0) |

$Q_0$ - Grenzwert für den Quotienten $Q$ ($Q_0 > 0$)

Eine vielfach anwendbare Konfiguration wäre etwa:

$$d_{max} = 2 \cdot d_0$$
$$n = 10$$
$$Q_0 = 20$$

Das erfindungsgemäße Verfahren ist in der Lage, bei geeigneter Konfiguration mit der technisch möglichen Wahrscheinlichkeit eine Fehlbestimmung des Rohwertes mit dem Operator "Schwellenwertmethode" zu vermeiden. Es liefert somit eine höhte Sicherheit in Bezug auf die korrekte Charakterisierung des Meßobjektes.

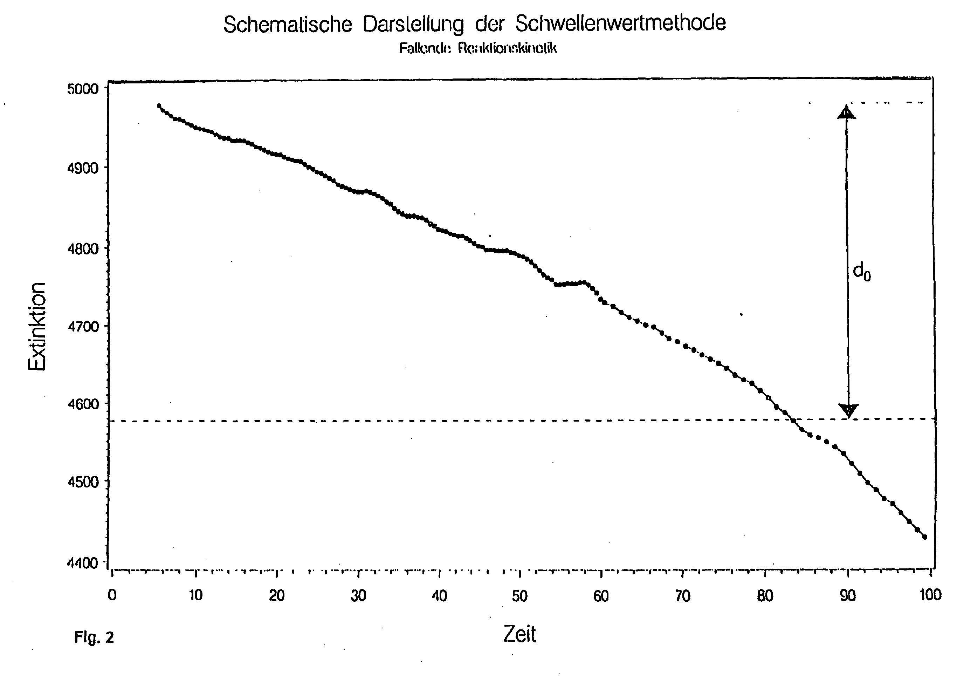

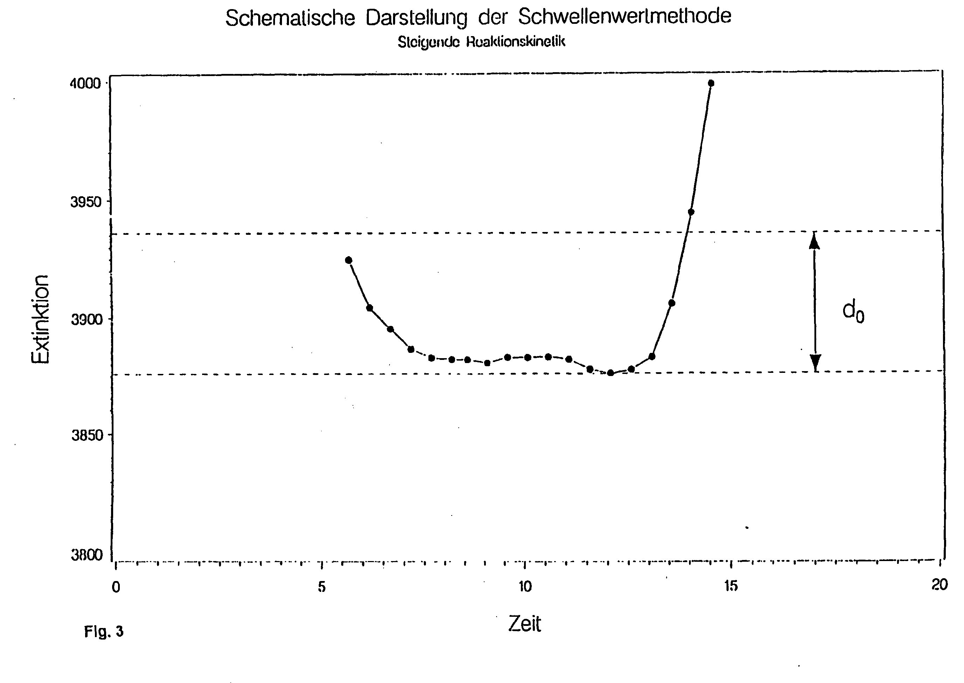

Das erfindungsgemäße Verfahren kann sowohl bei $d_0 > 0$ als auch $d_0 < 0$ angewendet werden. Im Falle $d_0 < 0$ (absteigende Kinetik, Fig. 2) gelten dieselben Randbedingungen wie bei $d_0 > 0$ (Fig. 3):

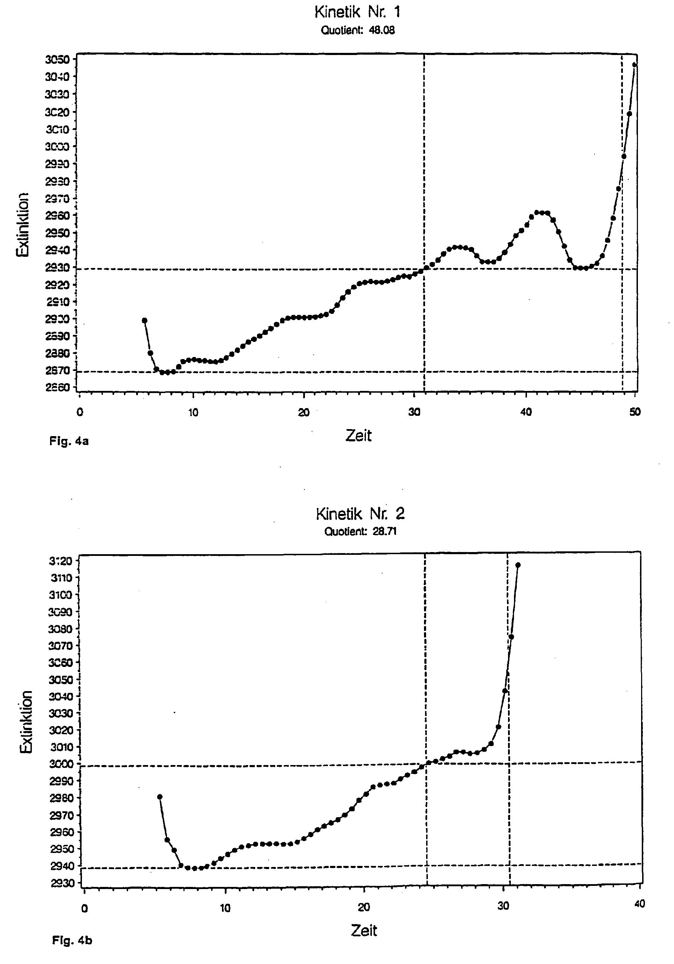

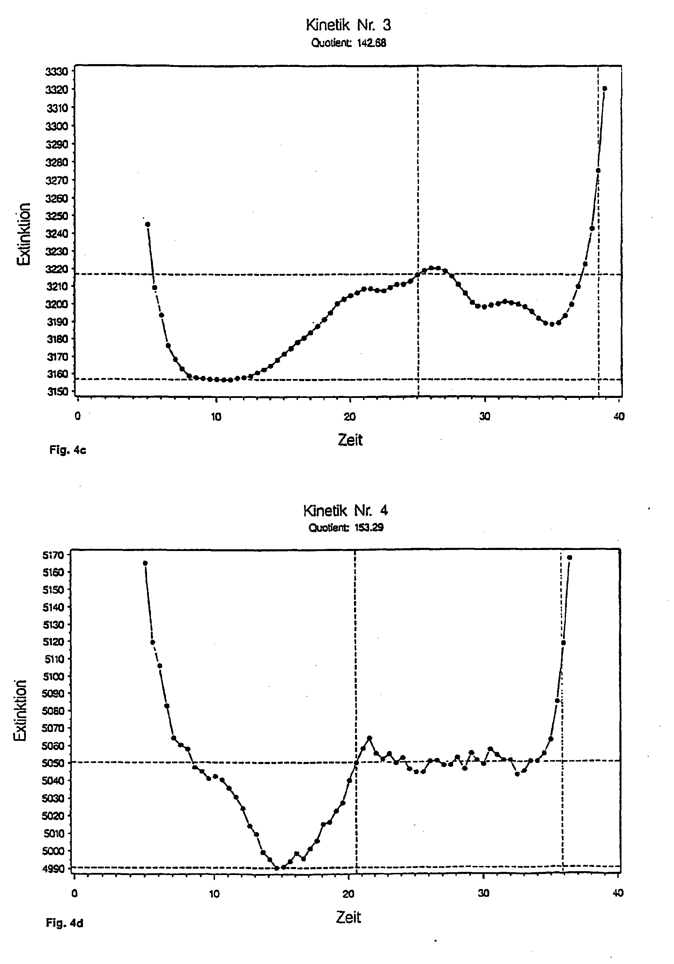

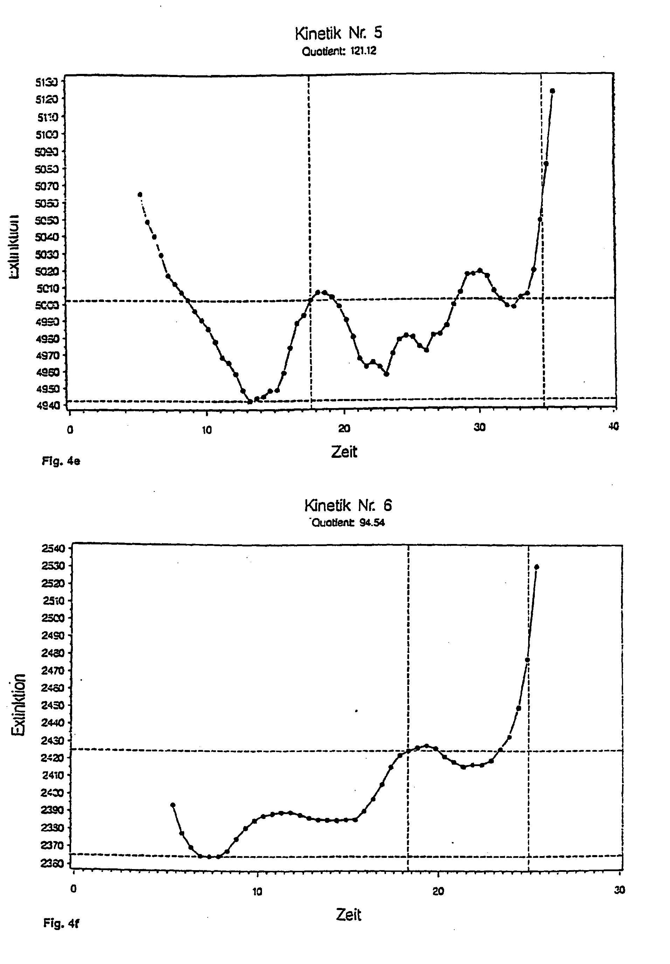

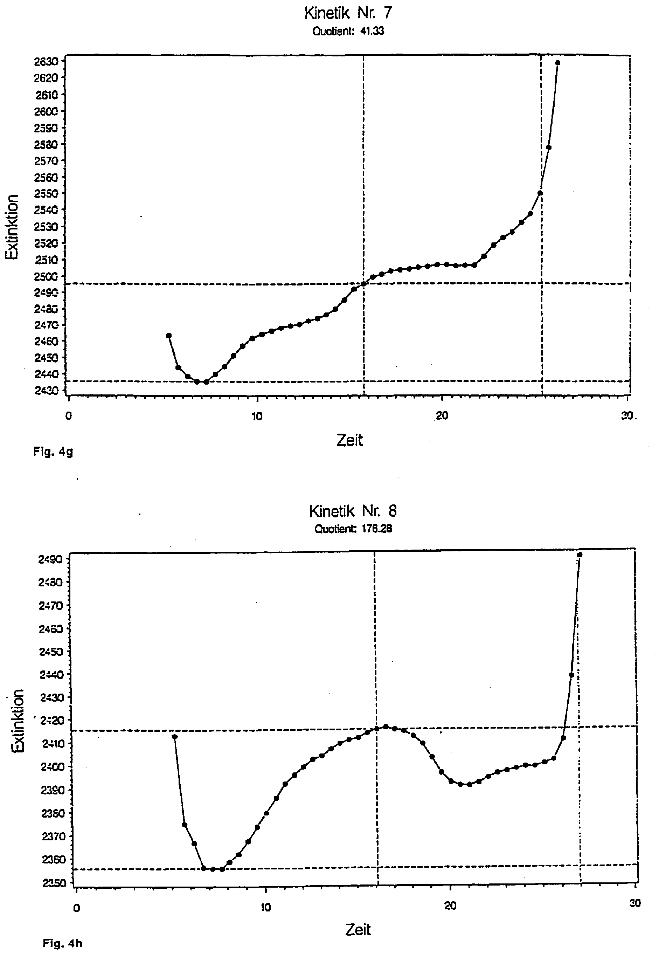

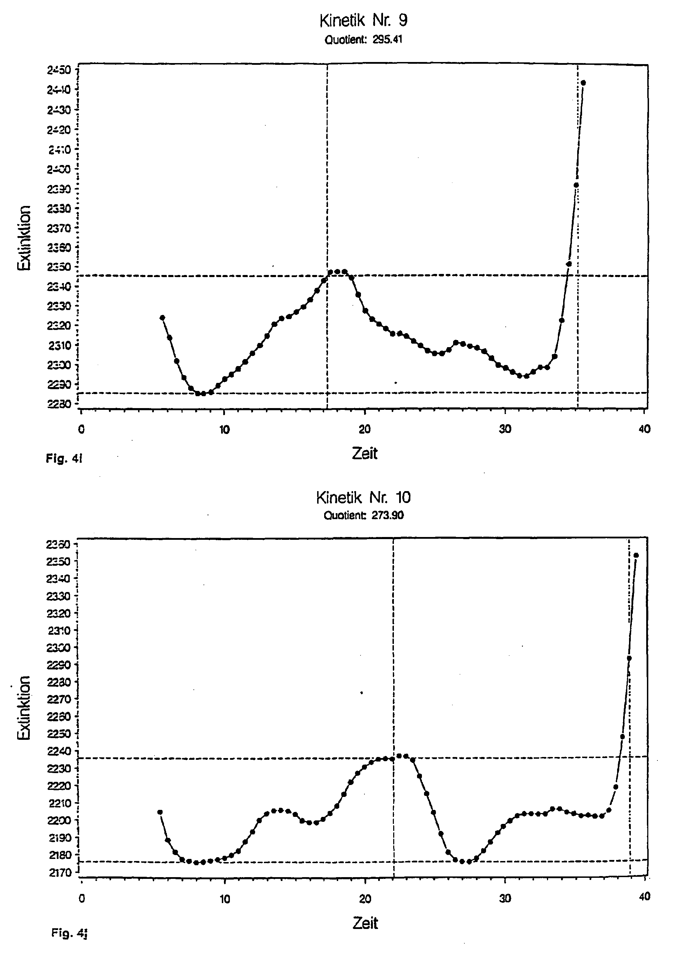

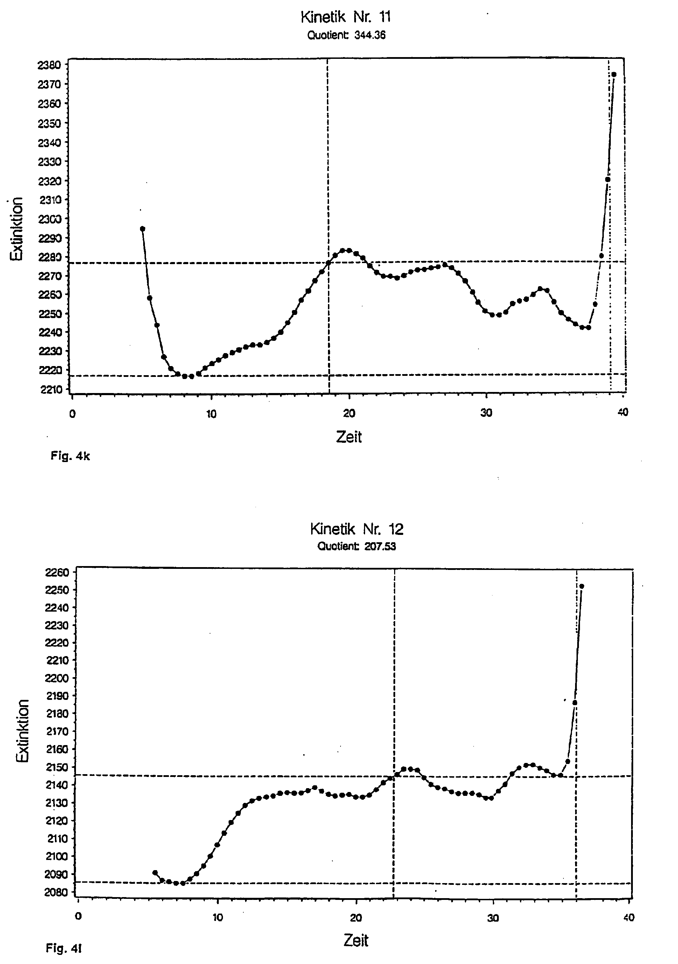

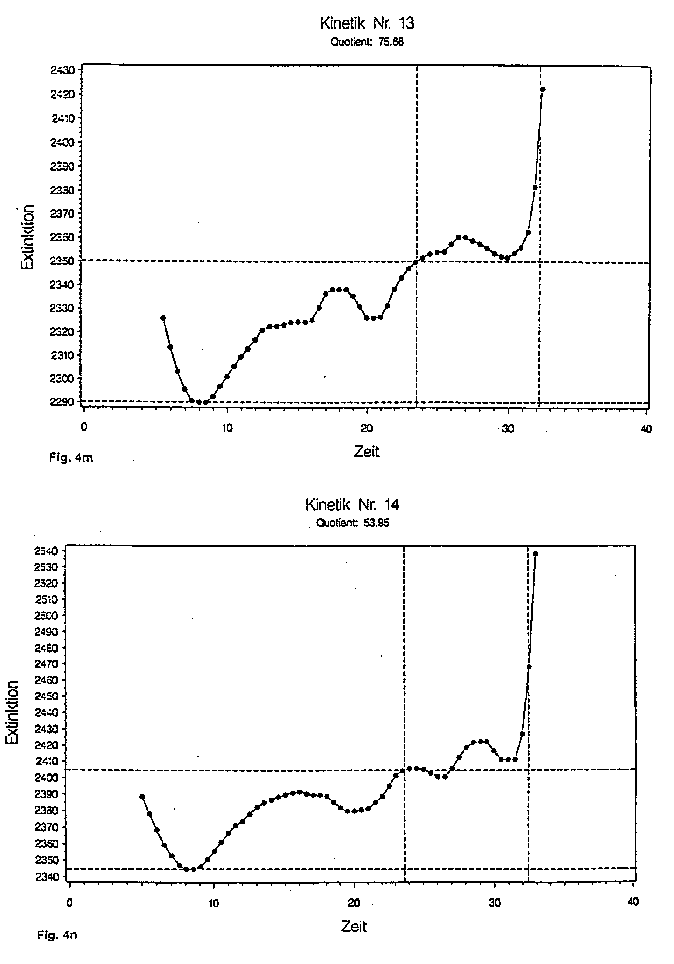

Die in den Figuren 1 bis 4 (a-n) dargestellten Beispiele sind ausschließlich dem Gebiet der Gerinnungsdiagnostik entnommen. Alle wurden nach der konventionellen Schwellenwertmethode unter Verwendung der Schwelle 60 falsch ausgewertet. Als Basiswert war dabei das Minimum des Kurvenverlaufs definiert. Im Titel der Graphik ist der jeweilige Quotient $Q$ angegeben, welcher sich unter der oben angegebenen typischen Konfiguration ergibt. Mit der dort angegebenen Grenze von 20 für den Quotienten konnten demnach alle Verläufe als kritisch eingestuft werden. Eine nachfolgende therapeutische Maßnahme auf der Basis der falschen Rohwerte hätte mit an Sicherheit grenzender Wahrscheinlichkeit zu lebensbedrohlichen Zuständen bei den entsprechenden Patienten geführt.

Das erfindungsgemäße Verfahren ist anwendbar auf alle Messungen eines im zeitlichen Verlauf sich ändemden Zustandes eines Meßobjektes, welcher durch eine steigende oder fallende Reaktionskurve charakterisiert wird und wobei die Reaktion dem Nachweis oder der Bestimmung eines biologischen Parameters oder Analyten dient und, wobei der Zeitpunkt von Interesse ist, zu dem das System einen vorgegebenen Kanal für den Kurvenverlauf verläßt.

Besonders vorteilhaft kann das erfindungsgemäße Verfahren im diagnostischen Verfahren angewendet werden. Als Beispiele wären hier zu nennen:

- Diagnostische Verfahren, bei denen der Zeitpunkt des Eintretens eines bestimmten Zustandes von Interesse ist (z. B. Bestimmung von Plasmaproteinen, enzymatische Reaktionen (z. B. ELISA))
- Chemische Reaktionsverläufe, bei denen der Zeitpunkt des Eintretens eines bestimmten Zustandes von Interesse ist

Die Figuren zeigen die Ergebnisse einer Serie von PT (Prothrombinzeit)-Bestimmungen verschiedener Patientenseren. Die linke gestrichelte vertikale Linie liefert den Rohwert (Schnittpunkt mit der x-Achse), welcher mit der konventionellen Schwellenwertmethode erhalten würde. Die rechte gestrichelte vertikale Linie zeigt den korrekten Rohwert für die jeweilige Kinetik an.

Beispiele:

Es wurden bei 14 Patentenplasmen aus einem klinischen Kollektiv die Prothrombinzeiten bestimmt. Es wurde das Prothrombinreagenz Thromborel S (Behring Diagnostics GmbH, Best. Nr. OUHP) an dem Gerinnungsauto BCS (Behring Coagulation System, Behring Diagnostics GmbH) eingesetzt. Das Reagenz wurde gemäß den Angaben des Herstellers eingesetzt.

**Patentansprüche**

1. Instrument zur Auswertung von Reaktionskinetiken, wobei die Reaktion dem Nachweis oder der Bestimmung eines biologischen Parameters oder Analyten dient, **dadurch gekennzeichnet, daß** ein Verfahren zur störungsfreien Auswertung von Reaktionskinetiken nach der Schwellenwertmethode, bei der eine Reaktionskinetik **dadurch** ausgewertet wird, daß die Veränderung einer reaktionsabhängigen Meßgröße (S) gemessen und die Zeit (T) bis zur erstmaligen Überschreitung eines Grenzwertes gemessen wird, hard- oder softwaremäßig installiert ist und wobei folgende Schritte durchgeführt werden:

   a) es werden für die Reaktion spezifische Werte für einen initialen Wert $d_0$, für einen maximalen Wert $d_{max}$ ($> d_0$), sowie eine Schrittzahl n und **dadurch** eine Schrittweite $d_s = (d_{max}-d_0)/n$ sowie ein Grenzwert $Q_0$ festgelegt,
   b) für den Bereich von d= $d_0$ bis $d_{max}$ wird jeweils für $d_n=(d_0+(d_s)*n)$ der Wert $T_n$ bestimmt,
   c) aus der geordneten Reihe der Werte $T_0$ bis $T_{max}$ werden die Differenzen $D_i = T_i - T_{i-1}$ für i = 1,...,n gebildet und aus der Menge $D_1$,..., $D_n$ der größte Wert $D_{max}$ und der kleinste Wert $D_{min}$ bestimmt, sowie der Quotient $Q=D_{max}/D_{min}$ gebildet,
   d) nur Messungen, für die $Q \leq Q_0$ gilt, werden zur weiteren Auswertung verwendet, Messungen, für die $Q > Q_0$ gilt, werden als zu wellig verworfen.

2. Instrument gemäß Anspruch 1 **dadurch gekennzeichnet, daß** auch als wellig erkannte Messungen ausgewertet werden, indem als gültiges Meßergebnis die zu $d_{max}$ gehörige Zeit $T_{max}$ genommen wird.

3. Instrument gemäß Anspruch 1 oder 2, wobei die Reaktion dem Nachweis oder der Bestimmung eines Parameters aus der Gerinnung dient.

**4.** Instrument gemäß Anspruch 3, wobei der Parameter die Prothrombinzeit ist.

**5.** Verfahren zur Bestimmung von Gerinnungsparametern, in dem eine Probe eines biologischen Materials mit einem Reaktionspartner versetzt wird, der zu einer parameterabhängigen Veränderung einer Meßgröße (S) führt **dadurch gekennzeichnet, daß** die Reaktionskinetik nach einem Verfahren zur störungsfreien Auswertung von Reaktionskinetiken nach der Schwellenwertmethode ausgewertet wird, bei der eine Reaktionskinetik **dadurch** ausgewertet wird, daß die Veränderung einer reaktionsabhängigen Meßgröße (S) gemessen und die Zeit (T) bis zur erstmaligen Überschreitung eines Grenzwertes gemessen wird, und wobei folgende Schritte durchgeführt werden:

a) es werden für die Reaktion spezifische Werte für einen initialen Wert $d_0$, für einen maximalen Wert $d_{max}$ ($> d_0$ ), sowie eine Schrittzahl n und **dadurch** eine Schrittweite $d_s = (d_{max}-d_0)/n$ sowie ein Grenzwert $Q_0$ festgelegt,
b) für den Bereich von d= $d_0$ bis $d_{max}$ wird jeweils für $d_n=(d_0+(d_s)*n)$ der Wert $T_n$ bestimmt,
c) aus der geordneten Reihe der Werte $T_0$ bis $T_{max}$ werden die Differenzen $D_i =T_i -T_{i-1}$ für i = 1,...,n gebildet und aus der Menge $D_1$,..., $D_n$ der größte Wert $D_{max}$ und der kleinste Wert $D_{min}$ bestimmt, sowie der Quotient $Q=D_{max}/D_{min}$ gebildet,
d) nur Messungen, für die $Q \le Q_0$ gilt, werden zur weiteren Auswertung verwendet, Messungen, für die $Q > Q_0$ gilt, werden als zu wellig verworfen.

**6.** Verfahren zur Bestimmung von Gerinnungsparametern gemäß Anspruch 5 **dadurch gekennzeichnet, daß** auch als wellig erkannte Messungen ausgewertet werden, indem als gültiges Meßergebnis die zu $d_{max}$ gehörige Zeit $T_{max}$ genommen wird.

**Claims**

**1.** An instrument for the evaluation of reaction kinetics, the reaction being used to detect or determine a biological parameter or analyte, wherein a method for the accurate evaluation of reaction kinetics using the threshold-value method, in which the kinetics of a reaction are evaluated by measuring the change in a variable (S) which depends on the reaction, and measuring the time (T) taken for a limit value to be exceeded for the first time, is installed at the hardware or software level and where the following steps are carried out:

a) values, specific to the reaction, of an initial value $d_0$ and of a maximum value $d_{max}$ ($> d_0$) and also a step number n and hence a step size $d_s = (d_{max}-d_0)/n$ and also a limit value $Q_0$ are established,
b) for the range from d = $d_0$ to $d_{max}$, the value $T_n$ is determined for each $d_n$ $(d_0+ (d_s)*n)$,
c) from the ordered series of values $T_0$ to $T_{max}$, the differences $D_i=T_i-T_{i-1}$ are taken for i=1, ... , n, and the largest value $D_{max}$ and the smallest value $D_{min}$ from the set $D_1$,..., $D_n$ are determined, and also the ratio $Q=D_{max}/D_{min}$ is taken,
d) only measurements for which $Q \le Q_0$ are used for further evaluation, measurements for which $Q > Q_0$ being discarded as too inconstant.

**2.** The instrument as claimed in claim 1, wherein measurements deemed inconstant are also evaluated by taking the time $T_{max}$ associated with $d_{max}$ as the current measurement result.

**3.** The instrument as claimed in claim 1 or 2, the reaction being used to detect or determine a coagulation parameter.

**4.** The instrument as claimed in claim 3, the parameter being the prothrombin time.

**5.** A method for determining coagulation parameters, by adding a reaction partner, which leads to a parameter-dependent change in a variable (S), to a sample of a biological material, wherein the reaction kinetics are evaluated using a method for the accurate evaluation of reaction kinetics using the threshold-value method, in which the kinetics of a reaction are evaluated by measuring the change in a variable (S) which depends on the reaction, and measuring the time (T) taken for a limit value to be exceeded for the first time, and where the following steps are carried out:

a) values, specific to the reaction, of an initial value $d_0$ and of a maximum value $d_{max}$ ($> d_0$) and also a step number n and hence a step size $d_s = (d_{max}-d_0)/n$ and also a limit value $Q_0$ are established,
b) for the range from d = $d_0$ to $d_{max}$, the value $T_n$ is determined for each $d_n=(d_0+(d_s)*n)$,
c) from the ordered series of values $T_0$ to $T_{max}$, the differences $D_i=T_i-T_{i-1}$ are taken for i=1, ... , n, and the largest value $D_{max}$ and the smallest value $D_{min}$ from the set $D_1$,..., $D_n$ are determined, and also the ratio $Q=D_{max}/D_{min}$ is taken,
d) only measurements for which $Q \le Q_0$ are used for further evaluation, measurements for which $Q > Q_0$ being discarded as too inconstant.

**6.** The method for determining coagulation parameters as claimed in claim 5, wherein measurements deemed inconstant are also evaluated by taking the time $T_{max}$ associated with $d_{max}$ as the current measurement result.

## Revendications

**1.** Instrument pour l'évaluation de cinétiques réactionnelles, dans lequel la réaction sert à détecter ou à déterminer un paramètre biologique ou un analyte, **caractérisé en ce que** l'on installe, sous la forme d'un équipement informatique ou d'un logiciel, un procédé d'évaluation sans perturbations de cinétiques réactionnelles selon le procédé de la valeur de seuil, dans lequel on évalue une cinétique réactionnelle en mesurant la variation d'une grandeur de mesure (S) dépendant de la réaction et le temps (T) qui s'écoule jusqu'au premier dépassement d'une valeur limite, et dans lequel on réalise les étapes suivantes :

a) on définit des valeurs spécifiques à la réaction pour une valeur initiale $d_0$, pour une valeur maximale $d_{max}$ (> $d_0$), ainsi qu'un nombre d'étapes n et donc un incrément $d_s = (d_{max} - d_0)/n$ ainsi qu'une valeur limite $Q_0$,
b) pour la plage de d = $d_0$ à $d_{max}$, on définit respectivement pour $d_n = (d_0 + (d_s)*n)$ la valeur $T_n$,
c) à partir de la série ordonnée des valeurs $T_0$ à $T_{max}$, on forme les différences $D_i = T_i - T_{i-1}$ pour i = 1, ..., n et, à partir de la quantité $D_1$ ..., $D_n$, on définit la plus grande valeur $D_{max}$ et la plus petite valeur $D_{min}$ ainsi que le quotient Q = $D_{max}/D_{min}$,
d) seules les mesures pour lesquelles Q ≤ $Q_0$ sont utilisées pour l'autre évaluation, tandis que les mesures pour lesquelles Q > $Q_0$ sont rejetées comme étant trop ondulatoires.

**2.** Instrument selon la revendication 1, **caractérisé en ce que** l'on évalue également les mesures reconnues comme ondulatoires en prenant comme résultat de mesure valable le temps $T_{max}$ correspondant à $d_{max}$.

**3.** Instrument selon la revendication 1 ou 2, dans lequel la réaction sert à détecter ou à déterminer un paramètre à partir de la coagulation.

**4.** Instrument selon la revendication 3, dans lequel le paramètre est le temps de prothrombine.

**5.** Procédé pour déterminer des paramètres de coagulation, dans lequel on mélange un échantillon d'un matériau biologique à un partenaire réactionnel, ce qui conduit à une variation dépendant des paramètres d'une grandeur de mesure (S), **caractérisé en ce que** la cinétique réactionnelle est évaluée selon un procédé d'évaluation sans perturbation de cinétiques réactionnelles suivant le procédé de la valeur de seuil, dans lequel on évalue une cinétique réactionnelle en mesurant la variation d'une grandeur de mesure (S) dépendant de la réaction et le temps (T) jusqu'au premier dépassement d'une valeur limite, et dans lequel on effectue les étapes suivantes :

a) on définit des valeurs spécifiques à la réaction pour une valeur initiale $d_0$, pour une valeur maximale $d_{max}$ (> $d_0$), ainsi qu'un nombre d'étapes n et donc un incrément $d_s = (d_{max} - d_0)/n$ ainsi qu'une valeur limite $Q_0$,
b) pour la plage de d = $d_0$ à $d_{max}$, on définit respectivement pour $d_n = (d_0 + (ds) * n)$ la valeur $T_n$,
c) à partir de la série ordonnée des valeurs $T_0$ à $T_{max}$, on calcule les différences $D_i = T_i - T_{i-1}$ pour i = 1, ..., n et à partir de la quantité $D_1$, ..., $D_n$, on définit la plus grande valeur $D_{max}$ et la plus petite valeur $D_{min}$ ainsi que le quotient Q = $D_{max}/D_{min}$),
d) seules les mesures pour lesquelles Q ≤ $Q_0$, sont utilisées pour l'autre évaluation, tandis que les mesures pour lesquelles Q > $Q_0$ sont rejetées comme étant trop ondulatoires.

**6.** Procédé pour déterminer des paramètres de coagulation selon la revendication 5, **caractérisé en ce que** l'on évalue également les mesures reconnues comme ondulatoires en prenant comme résultat de mesure valable le temps $T_{max}$ correspondant à $d_{max}$.

Fig. 1

Schematische Darstellung der Schwellenwertmethode
Fallende Reaktionskinetik

Fig. 2

EP 0 861 687 B1

Schematische Darstellung der Schwellenwertmethode
Steigende Reaktionskinetik

Fig. 3

EP 0 861 687 B1

## Kinetik Nr. 1
### Quotient: 48.08

Fig. 4a

## Kinetik Nr. 2
### Quotient: 28.71

Fig. 4b

Kinetik Nr. 3
Quotient: 142.68

Fig. 4c

Kinetik Nr. 4
Quotient: 153.29

Fig. 4d

### Kinetik Nr. 5
Quotient: 121.12

Fig. 4e

### Kinetik Nr. 6
Quotient: 94.54

Fig. 4f

**Kinetik Nr. 7**
Quotient: 41.33

Fig. 4g

**Kinetik Nr. 8**
Quotient: 176.28

Fig. 4h

## Kinetik Nr. 9
### Quotient: 295.41

**Fig. 4i**

## Kinetik Nr. 10
### Quotient: 273.90

**Fig. 4j**

Kinetik Nr. 11
Quotient: 344.36

Fig. 4k

Kinetik Nr. 12
Quotient: 207.53

Fig. 4l

14

## Kinetik Nr. 13
### Quotient: 75.66

Fig. 4m

## Kinetik Nr. 14
### Quotient: 53.95

Fig. 4n

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **von KOLDE.** *Behring Inst. Mitt.,* 1985, vol. 78, 176-187